(19) Europäisches Patentamt
European Patent Office
Office européen des brevets

(11) Veröffentlichungsnummer: **0 123 883 B1**

(12) **EUROPÄISCHE PATENTSCHRIFT**

(45) Veröffentlichungstag der Patentschrift: **18.09.91**

(51) Int. Cl.[5]: **C12Q 1/56**, G01N 33/86

(21) Anmeldenummer: **84103163.6**

(22) Anmeldetag: **22.03.84**

(54) **Verfahren zur fotometrischen Bestimmung der aktivierten partiellen Thromboplastinzeit und Reagenz dazu.**

(30) Priorität: **28.03.83 DE 3311287**

(43) Veröffentlichungstag der Anmeldung:
**07.11.84 Patentblatt 84/45**

(45) Bekanntmachung des Hinweises auf die Patenterteilung:
**18.09.91 Patentblatt 91/38**

(84) Benannte Vertragsstaaten:
**AT BE CH DE FR GB IT LI LU NL SE**

(56) Entgegenhaltungen:
**EP-A- 0 020 895**
**EP-A- 0 034 122**
**US-A- 3 486 981**

**CHEMICAL ABSTRACTS, Band 96, Nr. 5, 1. Februar 1982, Seite 417, Zusammenfassung Nr. 32381y, Columbus, Ohio, US; G. TANS et al.: "Properties of sulfatides in factor-XII-dependent contact activation", & BLOOD 1982, 59(1), 69-75 (Kat. D)**

**Idem**

(73) Patentinhaber: **BEHRINGWERKE Aktiengesellschaft**
**Postfach 1140**
**W-3550 Marburg 1(DE)**

(72) Erfinder: **Kolde, Hans-Jürgen, Dr.**
**Höhenweg 54**
**W-3550 Marburg(DE)**

(74) Vertreter: **Meyer-Dulheuer, Karl-Hermann, Dr. et al**
**HOECHST Aktiengesellschaft Postfach 3540**
**W-6200 Wiesbaden(DE)**

**EP 0 123 883 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 97, Nr. 13, 27. September 1982, Seite 419, Zusammenfassung Nr. 107863p, Columbus, Ohio, US; S. OHISHI: "Activation of factor XII in several congenitally deficient plasmas. Some aspects of activators dependent or independent of HMW-kininogen and prekallikrein", & AGENTS ACTIONS SUPPL. 1982, AAS 9(Recent Prog. Kinins), 26-30

CHEMICAL ABSTRACTS, Band 98, Nr. 23, 6. Juni 1983, Seite 429, Zusammenfassung Nr. 195638t, Columbus, Ohio, US; T. SHIMADA et al.: "Role of HMW kininogen in surface-mediated activation of factor XII", & ADV. EXP. MED. BIOL. 1983, 156A(Kinins-3, Pt. A), 73-85

BIOCHEMISTRY, Band 19, Nr. 7, 1980, Seiten 1322-1330, American Chemical Society, Easton, PA, US; K. FUJIKAWA et al.: "Activation of bovine factor XII (Hageman factor) by plasma kallikrein"

BIOCHEMISTRY, Band 19, Nr. 7, 1980, Seiten 1330-1346, American Chemical Society, Easton, PA, US; K. KURACHI et al.: "Mechanism of activation of bovine factor XI by factor XII and factor XIIa"

CLINICAL CHEMISTRY, Band 26, Nr. 10, September 1980, Easton, PA, US; J. FAREED et al.: "New perspectives in coagulation testing"

## Beschreibung

Die Erfindung betrifft ein Verfahren zur fotometrischen Bestimmung der aktivierten partiellen Thromboplastinzeit (APTT) sowie ein dazu geeignetes Reagenz.

Die Bestimmung der aktivierten partiellen Thromboplastinzeit (APTT) ist neben der Thromboplastinzeit (Prothrombinzeit, Quick-Wert) der am häufigsten durchgeführte Gerinnungstest. Die APTT erlaubt Aussagen über das Verhalten des endogenen Weges der Gerinnung. Eine weitere wichtige Anwendung dieses Tests liegt in der Kontrolle der Heparintherapie. Die APTT erfaßt das hochmolekulare Kininogen, Präkallikrein und die Faktoren XII, X, IX, VIII, V und I, daneben auch die Inhibitoren der Gerinnung, insbesondere Antithrombin III bei der Heparintherapie und Fibrinogenspaltprodukte (Antithrombin VI), die alle die APTT verlängern.

Reagenzien für die Bestimmung der APTT enthalten im wesentlichen Phospholipide und einen geeigneten Aktivator der "Kontaktphase". Durch die Kontaktaktivierung wird der Faktor XII aktiviert, der dann Faktor XI und Präkallikrein aktiviert. Durch die im Reagenz enthaltenen Lipide und Calciumionen kommt es dann zu einer Aktivierung des gesamten endogenen Weges, die in einer Fibringerinnselbildung endet. Die bis zu diesem Zeitpunkt benötigte Zeit ist Meßparameter.

Als Aktivatoren der Kontaktphase werden anorganische Materialien, vorzugsweise Celite oder Kaolin eingesetzt. Daneben findet auch Ellagsäure (US-P 3,486,981 und DE-OS 29 15 310) Verwendung. Ellagsäure bietet als optisch klares Reagenz Vorteile bei der Verwendung optischer Meßverfahren zur Detektion der Gerinnselbildung. Nach Bock et al., Biochemistry 20, 7258-7266 (1982) soll allerdings die wirksame Spezies ein wasserunlöslicher Komplex aus Metallionen und Ellagsäure sein. Ein weiterer Aktivator ist Dextransulfat.

Diese Aktivatoren der Kontaktphase sind jedoch unphysiologisch und auch schwierig zu standardisieren. Beispielsweise hängt die Gerinnungszeit einer kaolinaktivierten APTT ganz wesentlich von der Partikelgröße des Aktivators ab. Auch werden einige Gerinnungsfaktoren derartig fest an solche oberflächenaktive Materialien adsorbiert, daß man diese als Adsorbenzien zur Präparation der Kontaktfaktoren benutzt. Daneben ist aber auch die Zusammensetzung des Phospholipids, das ein solches Reagenz enthält, wesentlich für das Ergebnis. Auch die Länge der Vorinkubationszeit, nach der die eigentliche Reaktion, die zur Gerinnselbildung führt, durch "Rekalzifizierung" gestartet wird, spielt eine wichtige Rolle, da aktivierte Gerinnungsfaktoren durch die plasmatischen Inhibitoren, insbesondere Antithrombin III, gehemmt werden.

Als physiologische Aktivatoren der Gerinnung gelten Sulfatide (Fujikawa et al., Biochem. 19 (1980), 1322-1330 und Tans und Griffin, Blood, 59 (1982), 69-75. Sulfatide gehören zur Verbindungsklasse der Glycosphingolipide und enthalten am Galaktosering eine Sulfatgruppe. Zu dieser Verbindungsgruppe gehören unterschiedliche Spezies, die in der Art der Fettsäurekette differieren. Sie sind in allen Geweben in den Membranen nachweisbar, in besonders reichlicher Menge im Gehirn, aus dem man es auch in sehr reiner Form gewinnen kann. Sulfatide sind wirksamer als Kaolin bei der Kontaktaktivierung von Plasma.

Konventionelle APTT-Bestimmungen laufen auf die Messung eines Fibringerinnsels hinaus. Die Bildung eines solchen Gerinnsels ist meßtechnisch nur schwierig zu verfolgen. Eine ganze Reihe von Geräten wurde entwickelt, die unterschiedliche mechanische, elektrische oder optische Verfahren zu benutzen.

Seit Einführung chromogener Substrate für Gerinnungsfaktoren wurde auch versucht, diese für die Bestimmung der Gerinnungsenzyme einzusetzen. Der Vorteil chromogener Substrate liegt in der einfachen Standardisierbarkeit der niedermolekularen Substrate im Gegensatz zu den komplexen, hochmolekularen natürlichen Substraten. Es entfällt das Problem, die Bildung eines Gerinnsels zu messen.

Auch für die Durchführung von "Globaltests" wurden bereits chromogene Substrate eingesetzt. So beschrieben Yamada und Meguro, Thrombos. Res. 15 (1979), 351-358, eine Methode zur Durchführung der APTT. Diese beruht auf der Aktivierung des endogenen Weges mit Ellagsäure in Gegenwart von Phospholipid, Calcium und dem chromogenen Thrombin-Substrat H-D-Phe-Pip-Arg-pNA (S 2238). Nachteile dieses Verfahrens sind der Einsatz eines unphysiologischen Aktivators, die mangelhafte Spezifität des chromogenen Substrates und die extrem langen Meßzeiten (Normalwert um 7,4 Minuten). Über die Einzelfaktorenempfindlichkeit wurden nur unvollständige Daten publiziert.

Eine weitere Methode wird von P. Aiyappa, Ann. New York Acad. Sci. (1981), S. 812-821, beschrieben. In diesem Meßsystem wird Plasma durch Ellagsäure in Gegenwart von Phospholipid und Calciumionen 5 Minuten aktiviert und das bis dahin gebildete Thrombin mit einem chromogenen Substrat gemessen. Diese Methode kann zur Bildung von Fibrin führen, das aktives Thrombin einschließt. Auch kann Thrombin durch Inhibition wieder desaktiviert werden. Andererseits lassen sich pathologische Plasmen innerhalb der gewählten Inkubationszeit möglicherweise gar nicht oder nur unvollständig aktivieren, obwohl sie eigentlich, wenn auch verzögert, dazu in der Lage sind. Das von Aiyappa beschriebene Verfahren erlaubt nicht, in Mangelplasmen eine Gerinnung zu messen.

Überraschend wurde nun gefunden, daß wesentliche Nachteile der beschriebenen APTT-Methoden mit chromogenen Substraten durch den Einsatz eines Sulfatids als physiologischen Aktivator in Verbindung mit

einem hochspezifischen Thrombinsubstrat beseitigt werden können.

Gegenstand der Erfindung ist deshalb ein Verfahren zur Bestimmung der aktivierten partiellen Thromboplastinzeit mit einem Aktivator, einem gerinnungsaktiven Phospholipid oder Phospholipidgemisch, Calciumionen und einem chromogenen Substrat für Thrombin, dadurch gekennzeichnet, daß der Aktivator ein Sulfatid oder Sulfatidgemisch ist.

Gegenstand der Erfindung ist weiterhin ein Reagenz zur Bestimmung der aktivierten partiellen Thrombinzeit, bestehend aus einem Sulfatid, einem Phospholipid, einem löslichen Calciumsalz und einem chromogenen Substrat in lyophilisierter Form.

Brauchbare Sulfatide sind kommerziell erhältlich, beispielsweise von den Firmen Serva, Sigma oder Supelco. Vorteilhaft werden solche Sulfatide verwendet, die in einer Dünnschichtchromatographie auf Kieselgel und einem Gemisch aus Chloroform/Methanol/Wasser 65:25:4 als Laufmittel einem $R_F$-Wert von 0,2 - 0,3, bevorzugt 0,25, und mit Chloroform/Methanol 40:15 einen solchen von 0,25 - 0,35, bevorzugt 0,31, haben. Solche Sulfatide können aber auch nach beschriebenen Verfahren, beispielsweise nach Hara und Radin, Anal. Biochem. 100, 364-370 (1979) oder aus Hirnacetontrockenpulver, gewonnen werden. Sinnvolle Testkonzentrationen liegen zwischen 0,1 und 50 $\mu$g/ml. Das Sulfatid kann zunächst in einer Konzentration von 0,01 g/l in 50 mMol/l HEPES-Puffer, pH 7,6, suspendiert und für die Bereitung des Reagenzes verwendet werden.

Als Phospholipid können die in konventionellen Reagenzien eingesetzten tierischen und pflanzlichen Lipide verwendet werden. Besonders vorteilhaft ist der Einsatz von Lipiden aus menschlichen Thrombozyten oder eines Extrakts aus menschlischer Plazenta. Wichtig ist, daß diese Lipide keine thromboplastische Aktivität zeigen, um nicht auch den exogenen Weg zu aktivieren.

Das chromogene Substrat für Thrombin muß spezifisch sein, um in diesem Test eingesetzt werden zu können, da ja Thrombin selektiv neben den anderen ebenfalls aktivierten Gerinnungsfaktoren gemessen werden soll. Chromozym(R) TH (Tos-Gly-Pro-Arg-pNA) und S 2160 (Bz-Phe-Val-Arg-pNA) sind relativ unspezifisch und zeigen auch andere Gerinnungsfaktoren an wie Kallikrein oder die Faktoren Xa und XIIa. Besser eignet sich S 2238 (HD-Phe-Pip-Arg-pNA), obgleich auch hier die Spezifität gegenüber den Kontaktfaktoren nicht sehr hoch ist. Außer Para-Nitroaniliden sind auch Peptide mit anderen Chromophoren geeignet, beispielsweise Cumarinderivate, wobei die Hydrolyse durch Messung der Fluoreszenz verfolgt wird.

Die für einen Globaltest wie der APTT am besten geeigneten chromogenen Peptide sind Thrombinsubstrate, wie sie in der deutschen Patentanmeldung P 32 44 030.8 beschrieben sind. Sie enthalten als Chromophor Derivate der 5-Amino-2-nitrobenzoesäure.

Bevorzugt wird eine Verbindung der allgemeinen Formel I

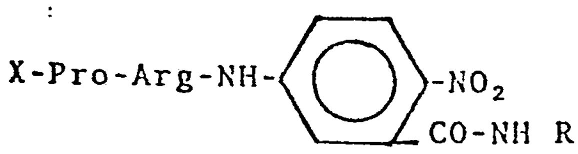

(I)

mit R = $C_{1-5}$-Alkyl oder $-CH[CH(CH_3)_2]COOCH_3$ und X = H-D-Phe-, BOC-Gly- oder Tosyl-Gly- verwendet.

Das Substrat wird vorzugsweise in sehr geringer Konzentration eingesetzt. Die besten Ergebnisse erzielt man mit 50 $\mu$Mol/l bei der Verwendung von H-D-Phe-Pro-Arg-ANBS-isopropylamid.Die Calciumkonzentration sollte zwischen 1 und 10 mMol/l betragen, bevorzugt werden 5 mMol/l.

Sulfatid, Phospholipid, chromogenes Substrat und das Calciumsalz, bevorzugt Calciumchlorid, werden in einem Puffer wie HEPES oder TRIS pH 7,2-8,5 gelöst oder suspendiert. Auch Imidazol-, Glycylglycin- oder Triethanolamin-Puffer sind brauchbar.

Das erfindungsgemäße Verfahren wird zweckmäßigerweise bei 25-37 °C ausgeführt, indem eine Plasmaprobe mit dem temperierten Reagenz in einer thermostatisierten Küvette zusammengebracht wird. In einem Photometer wird bei 405-410 nm die Reaktion verfolgt.

Bei einem typischen Normalplasma bleibt die Extinktion einige Zeit konstant, um dann innerhalb von wenigen Sekunden stark anzusteigen. Meßparameter ist die Zeit, die nötig ist, eine bestimmte Extinktionsdifferenz, beispielsweise 0,1, zu erreichen. Grundsätzlich ist auch eine kinetische Auswertung der exponentiellen Kurve möglich. Die Umsetzung einer bestimmten Substratmenge ist analog zu der einer konventionellen APTT, wo das aktivierte Thrombin eine bestimmte Menge Fibrinogen umsetzt und damit das Gerinnsel auslöst. Beispiel 1 zeigt, daß das neue Verfahren in der bevorzugten Form Änderungen der Aktivität jedes einzelnen Faktors des endogenen Weges anzeigt.

Im Gegensatz zur konventionellen APTT, in der nach einer bestimmten festen Aktivierungszeit die

eigentliche, zum Gerinnsel führende Reaktion durch $CaCl_2$-Zusatz gestartet wird, kann bei dem neuen Verfahren $CaCl_2$ von Anfang an zugesetzt werden, so daß man mit einem Pipettierschritt weniger auskommt (Monoreagenz). Prinzipiell ist jedoch auch ein dem bisherigen Verfahren analoges Testsystem mit dem erfindungsgemäßen Verfahren möglich. Zu diesem Zweck wird das chromogene Peptid zusammen mit $CaCl_2$ erst nach einer bestimmten Vorinkubationszeit zugesetzt. Es erweist sich dann als günstig, auch während der Vorinkubationsphase dem Sulfatidreagenz etwas $CaCl_2$ (etwa 1 mMol/l) zuzusetzen und die eigentliche Reaktion durch Zusatz von mehr $CaCl_2$-Lösung zu starten. Als Endkonzentration ist auch hier 5 mMol/l $CaCl_2$ optimal.

Die Verwendung von Sulfatiden zur Kontaktaktivierung von Plasma in Kombination mit chromogenen Substraten war bisher nicht bekannt. Reagenzienhersteller empfehlen die Lagerung der Sulfatide unter 0° C. Die Lyophilisation von Sulfatiden zusammen mit den notwendigen anderen Bestandteilen des Reagenzes führt zu keinem stabilen Produkt. Die APTT verlängert sich stets drastisch.

Überraschenderweise gelangt man durch Zusatz von Serumalbumin Gelatine oder einem abgebautem und vernetztem Kollagen zu einem stabilen, wirksamen Reagenz. Eine Konzentration von lediglich etwa 0,1-1% erlaubt die Herstellung eines Lyophilisates mit guter Aktivität.

Wie bei konventionellen Reagenzien ist die Verbindung mit Mangelplasma die Bestimmung der Einzelfaktoren des endogenen Weges möglich. Dazu wird die Plasmaprobe verdünnt werden, um den Einfluß der anderen Gerinnungsfaktoren auszuschalten oder sie wird im Unterschuß zu dem verwendeten Mangelplasma eingesetzt.

Ein Zusatz von Aminosäuren, besonders Glutamin, Asparagin oder Glutaminsäure, erhöht die Heparin-Empfindlichkeit des Reagenzes.

Abkürzungen:

| | |
|---|---|
| ANBS | 5-Amino-2-nitrobenzoesäure |
| Arg | Arginin |
| Gly | Glycin |
| HEPES | N-2-Hydroxyethylpiperazin-N'-2-ethansulfonsäure |
| Phe | Phenylalanin |
| Pip | Pipecolinsäure |
| Pro | Prolin |
| Tos | Toluolsulfonyl- |
| TRIS | Tris(hydroxymethyl)aminomethan |

Das folgende Beispiele erläutert die Erfindung.

Beispiel 1:

Faktorenempfindlichkeit des neuen Reagenzes

Reagenz:

| | |
|---|---|
| 0,1 ml | 3 mMol/l H-D-Phe-Pro-Arg-ANBS-isopropylamid Chromogenes Substrat (S 82 107) |
| 0,5 ml | Fibraccel® 1:1000 (gerinnungsaktiver homologer Phospholipidkomplex, Behringwerke AG) |
| 0,5 ml | Sulfatidlösung (Fa. Supelco; $R_F$ 0,25 mit $CHCl_3/MeOH/H_2O$ 65:25:4 auf Kieselgel) 0,01 g/l |
| 5,0 ml | Puffer (HEPES, NaCl, $Ca^{2+}$: 25, 50, 5 mMol/l; pH 7,6, 0,25 % Haemaccel® (DE-PS 11 18 792 und 11 53 134) |

Ansatz:

| | |
|---|---|
| 100 ul | Plasma bzw. Mangelplasma (Behringwerke), F VII-Mangelplasma kongenital |
| 1 ml | Reagenz |

37° C, Messung der Zeit, bis $E_{405\ nm}$ = 0,1.

| Mangelplasma | Sekunden |
|---|---|
| Faktor II | 1.200 |
| Faktor V | 1.200 |
| Faktor VII | 207 |
| Faktor VIII | 378 |
| Faktor IX | 498 |
| Faktor X | 448 |
| Faktor XI | 396 |
| Faktor XII | 336 |
| HMWK (Hochmolekulares Kininogen) | 396 |
| Kallikrein | 720 |
| Poolplasma | 170 |

**Patentansprüche**

1. Verfahren zur Bestimmung der aktivierten partiellen Thromboplastinzeit (APTT) mittels eines chromogenen Substrats und Aktivierung durch Sulfatide oder Sulfatidgemische, dadurch gekennzeichnet, daß, außer der Probe, alle für die Testdurchführung notwendigen Reagenzien in einem Schritt zugegeben werden.

2. Verfahren gemäß Anspruch 1, dadurch gekennzeichnet, daß das chromogene Sustrat eine Verbindung der allgemeinen Formel I ist:

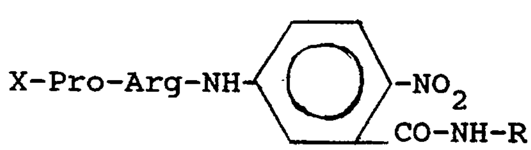

(I)

mit R = $C_{1-5}$-Alkyl oder $-CH[CH(CH_3)_2]COOCH_3$ und
X = H-D-Phe- oder Tosyl-Gly-
ist.

3. Ein Reagenz zur Bestimmung der APTT mittels eines Verfahrens gemäß Anspruch 1, dadurch gekennzeichnet, daß es alle für die Testdurchführung nötigen Bestandteile (ein Sulfatid oder Sulfatidgemisch, Phospholipid, Kalziumionen und ein chromogenes Thrombinsubstrat) in einer Lösung enthält.

4. Ein Reagenz gemäß Anspruch 3, dadurch gekennzeichnet, daß es einen Puffer enthält, vorzugsweise HEPES, pH 7.2 - 8.5.

5. Ein Reagenz gemäß Anspruch 3, dadurch gekennzeichnet, daß es zusätzlich eine Aminosäure, bevorzugterweise Glutamin, Asparagin oder Glutaminsäure, enthält.

6. Ein Reagenz gemäß Anspruch 3, dadurch gekennzeichnet, daß es zusätzlich Serumalbumin, Gelatine oder ein abgebautes und vernetztes Kollagen enthält.

7. Ein Reagenz gemäß Anspruch 3, dadurch gekennzeichnet, daß es in lyophilisierter Form vorliegt.

8. Verfahren gemäß Anspruch 1, dadurch gekennzeichnet, daß zur Bestimmung von Einzelfaktoren die Probe mit einem entsprechenden Mangelplasma verdünnt wird.

## Claims

1. A method for the determination of the activated partial thromboplastin time (APTT) by means of a chromogenic substrate and activation by sulfatides or mixtures of sulfatides, wherein all the reagents, apart from the sample, necessary for carrying out the test are added in one step.

2. The method as claimed in claim 1, wherein the chromogenic substrate is a compound of the formula I:

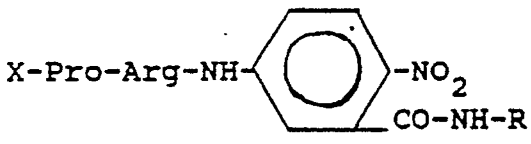

(I)

where R is $C_{1-5}$-alkyl or $-CH[CH(CH_3)_2]COOCH_3$, and
X is H-D-Phe- or tosyl-Gly-.

3. A reagent for the determination of the APTT by means of a method as claimed in claim 1, which contains all the components necessary for carrying out the test (a sulfatide or mixture of sulfatides, phospholipid, calcium ions and a chromogenic thrombin substrate) in one solution.

4. A reagent as claimed in claim 3, which contains a buffer, preferably HEPES, of pH 7.2 - 8.5.

5. A reagent as claimed in claim 3, which additionally contains an amino acid, preferably glutamine, asparagine or glutamic acid.

6. A reagent as claimed in claim 3, which additionally contains serum albumin, gelatin or a degraded and crosslinked collagen.

7. A reagent as claimed in claim 3, which is in freeze-dried form.

8. The method as claimed in claim 1, wherein the sample is diluted with an appropriate deficient plasma for determination of single factors.

## Revendications

1. Procédé pour déterminer le temps de thromboplastine partiel activé (TTPA) au moyen d'un substrat chromogène et avec activation par des sulfatides ou mélanges de sulfatides, caractérisé en ce que l'on ajoute, en une étape, tous les réactifs nécessaires à la conduite du test, en dehors de l'échantillon.

2. Procédé selon la revendication 1, caractérisé en ce que le substrat chromogène est un composé de formule générale I:

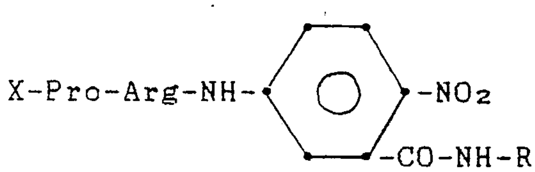

avec R = alkyle en $C_{1-5}$ ou $-CH[CH(CH_3)_2]COOCH_3$ et
X = H-D-Phe-, ou tosyl-Gly-.

3. Réactif pour la détermination du APTT au moyen d'un procédé selon la revendication 1, caractérisé en ce qu'il contient dans une solution tous les composants nécessaires à l'exécution du test (un sulfatide

ou mélange de sulfatides, un phospholipide, des ions calcium et un substrat chromogène pour la thrombine).

4. Réactif selon la revendication 3, caractérisé en ce qu'il contient un tampon, de préférence l'HEPES, à pH 7,2 - 8,5.

5. Réactif selon la revendication 3, caractérisé en ce qu'il contient de plus un acide aminé, au mieux la glutamine, l'asparagine ou l'acide glutamique.

6. Réactif selon la revendication 3, caractérisé en ce qu'il contient de plus de la sérumalbumine, de la gélatine ou un collagène dénaturé et réticulé.

7. Réactif selon la revendication 3, caractérisé en ce qu'il se présente sous forme lyophilisée.

8. Procédé selon la revendication 1, caractérisé en ce que, pour déterminer des facteurs isolés, on dilue l'échantillon avec un plasma déficient correspondant.